Europäisches Patentamt

**European Patent Office**

**Office européen des brevets**

(19)

(11) Veröffentlichungsnummer: **0 210 295**
**B1**

(12) **EUROPÄISCHE PATENTSCHRIFT**

(45) Veröffentlichungstag der Patentschrift:
**20.09.89**

(51) Int. Cl.⁴: **A61K 35/78**

(21) Anmeldenummer: **85109617.2**

(22) Anmeldetag: **31.07.85**

(54) **Arzneimittel zur Behandlung chronisch entzündlicher Darmerkrankungen.**

(43) Veröffentlichungstag der Anmeldung:
**04.02.87 Patentblatt 87/6**

(45) Bekanntmachung des Hinweises auf die Patenterteilung:
**20.09.89 Patentblatt 89/38**

(84) Benannte Vertragsstaaten:
**AT BE CH DE FR GB IT LI LU NL SE**

(56) Entgegenhaltungen:
**EP-A- 0 019 808**

**BIOLOGICAL ABSTRACTS, Band 62, 1976, Nr. 22342, Biological Abstracts Inc., Philadelphia, PA, US; R.J. ROBINS et al.: "The nature of the stimuli causing digestive juice secretion in Dionaea muscipula Ellis (Venus's flytrap)", & PLANTA (BERL) 128(3): 263-265, 1976 000**

(73) Patentinhaber: **Keller, Helmut, Dr.med., Blumenstrasse 32, D-8646 Nordhalben(DE)**

(72) Erfinder: **Keller, Helmut, Dr.med., Blumenstrasse 32, D-8646 Nordhalben(DE)**

(74) Vertreter: **Freiherr von Pechmann, Eckehart et al, Patentanwälte Wuesthoff- v. Pechmann-Behrens-Goetz Schweigerstrasse 2, D-8000 München 90(DE)**

## Beschreibung

Aus der EP 19 808 bzw. der entsprechenden Patentanmeldung 80 102 707 ist es bekannt, daß der Preßsaft bestimmter fleischfressender Pflanzen (Carnivoren) sich zur Krebsbekämpfung eignet. Als besonders wirksam haben sich die Inhaltsstoffe des Preßsaftes von Pflanzenteilen der Venusfliegenfalle (Dionaea muscipula) erwiesen. Diese Preßsäfte enthalten u.a. spezifische proteolytische Fermente und können oral oder nach Filtrieren und Verdünnen mit Kochsalzlösungen als Injektionspräparate appliziert werden. Wie in der Veröffentlichung: EHK Acta medica empirica, Bd. 34, 416-420 (1985) näher beschrieben ist, dürfte die Wirkung auch auf das Vorhandensein spezifischer Endonucleasen, die ebenfalls in den Preßsäften dieser fleischfressenden Pflanzen festgestellt wurden, zurückzuführen sein. Diese Wirkstoff bewirken eine Zellteilhemung bei den Tumorzellen, wodurch in vielen Fällen ein deutlicher Rückgang des Tumors bzw. sein Verschwinden erreicht werden konnte. Auch bei Patienten, die bereits Metastasen aufweisen, war die Therapie mit den im Handel unter der Bezeichnung CARNIVORA® der Firma Carnivora GmbH, Jagsthausen, erhältlichen Präparaten erfolgreich. Hierbei handelt es sich um verdünnte Preßsäfte oder um die lyophilisierten Feststoffe des Preßsaftes der frischen Pflanzenteile von Dionaea muscipula, die oral eingenommen oder nach Auflösen des lyophilisierten Pulvers in Wasser intramuskulär injiziert werden. Der Preßsaft kann auch nach Verdünnen mit Wasser in Form eines Aerosprays inhaliert werden.

Es wurde nun überraschenderweise festgestellt, daß diese Wirkstoffe des Preßsaftes der fleischfressenden Pflanzen sich auch zur Behandlung von chronisch entzündlichen Erkrankungen des Darms, nämlich bei Morbus Crohn und Colitis ulcerosa, eignen und zu Ausheilungen dieser bisher schwierig zu behandelnden, langwierigen Krankheiten führen. Dies ist deshalb so überraschend, weil es sich bei diesen Erkrankungen um Krankheiten ganz anderer Genese handelt als bei den Sarkomen oder Carzinomen, die bisher mit dem Preßsaft behandelt werden konnten.

Die granulomatös-entzündliche Erkrankung des Ileums und Colons, die als Enteritis regionalis oder Morbus Crohn bezeichnet wird, tritt häufig bereits in der Jugend auf. Sie ist verbunden mit chronischen Diarrhoen und Schmerzen in Abdomen. Die Therapie dieser Erkrankung ist meist nur symptomatisch, zwecks Minderung der Schmerzen und Diarrhoen. Durch antibakterielle Medikamente werden zwar bakterielle Komplikationen bekämpft, aber eine Heilung nicht erreicht. Corticosteride haben sich als nützlich erwiesen, doch ist häufig die operative Behandlung mit Resektion des betroffenen Darmabschnitts erforderlich, was auch für die chronische Colitis Ulcerosa gilt, wo bisher auch in schweren Fällen nur eint totale Proktokolektomie bzw. Ileostomie zur Heilung führte.

Erfindungsgemäß wird nun zur Herstellung eines Arzneimittels zur Behandlung von Morbus Crohn und der Colitis ulcerosa der Preßsaft der fleischfressenden Pflanzen verwendet. Als orale Applikationsform eignet sich auch hier der mit Wasser oder Kochsalzlösung verdünnte Saft der durch Auspressen der Pflanzen, insbesondere der Blätter, erhalten wird. Aber auch wäßrige Extrakte von Teilen der Carnivoren-Pflanzen eignen sich für die erfindungsgemäße Verwendung. Für Injektionszwecke wird der gefilterte Saft oder Extrakt im allgemeinen zunächst lyophilisiert und dann kurz vor der Applikation in Wasser aufgenommen und diese Lösung intramuskulär injiziert.

Vorzugsweise wird auch bei der neuen Indikation für die Wirkstoffe im Preßsaft von fleischfressenden Pflanzen der Saft der Blätter bzw. der ganzen Pflanze von Dionaea muscipula verwendet und das Arzneimittel zur Behandlung der chronischen Entzündungen des Darms dann oral oder intramuskulär appliziert.

### Beispiel 1

Zur Herstellung des injizierbaren Arzneimittels für die Behandlung von Morbus Crohn werden die frisch geernteten Pflanzenteile von Dionaea muscipula in üblicher Weise ausgepreßt, der Saft gefiltert und lyophilisiert, wobei 200 ml Preßsaft ca. 4,2 g Lyophilisat ergeben. Für die einzelne Injektion werden dann 41,5 mg des Lyophilisats in Ampullen abgefüllt. Nach Auflösen in Wasser wird das Arzneimittel intramuskulär in einer Dosierung, die etwa 2 ml Preßsaft entspricht, injiziert.

### Beispiel 2

Zur Herstellung eines oral anwendbaren Arzneimittels zur Behandlung der chronischen Darmerkrankungen wird der gefilterte Preßsaft nach Beispiel 1 mit alkoholhaltigem Wasser oder Kochsalzlösung im Verhältnis von 1 : 3 verdünnt. Hiervon sollen mehrmals täglich ca. 2 ml (50 Tropfen) oral eingenommen werden.

### Patentansprüche

1. Verwendung des Preßsaftes oder wäßrigen Extraktes fleischfressender Pflanzen zur Herstellung eines Arzneimittels zur Behandlung der chronisch entzündlichen Darmerkrankungen, nämlich Morbus Crohn und Colitis ulcerosa.

2. Verwendung des lyophilisierten Preßsaftes oder Extraktes der fleischfressenden Pflanzen zur Herstellung eines Arzneimittels zur Behandlung nach Anspruch 1.

3. Verwendung des Preßsaftes oder Extraktes oder des lyophilisierten Preßsaftes oder Extraktes der Pflanze Venusfliegenfalle (Dionaea muscipula) zur Herstellung eines Arzneimittels zur Behandlung nach Anspruch 1.

### Claims

1. Use of the pressed juice or aqueous extract of carnivorous plants for the production of a medicament for the treatment of the chronically inflamed intestinal diseases, namely morbus Crohn and colitis ulcerosa.

2. Use of the lyophilised pressed juice or extract of the carnivorous plants for the production of a medicament for the treatment according to claim 1.

3. Use of the pressed juice or extract or of the lyophilised pressed juice or extract of the plant Venus fly trap (Dionaea muscipula) for the production of a medicament for the treatment according to claim 1.

**Revendications**

1. Utilisation du jus de pression ou de l'extrait aqueux de plantes carnivores pour la fabrication d'un médicament pour le traitement des maladies chroniquement inflammatoires de l'intestin, à savoir la maladie de Crohn (Morbus Crohn) et la colite ulcéreuse (Colitis ulcérosa).

2. Utilisation du jus de pression ou de l'extrait lyophilisé de plantes carnivores pour la fabrication d'un médicament pour le traitement selon la revendication 1.

3. Utilisation du jus de pression ou de l'extrait ou du jus de pression ou de l'extrait lyophilisé de la plante Dionée Gobe-Mouches (Dionaea muscipula) pour la fabrication d'un médicament pour le traitement selon la revendication 1.